# EUROPEAN PATENT SPECIFICATION

(11) **EP 1 860 102 B1**
(45) Date of publication and mention of the grant of the patent: **26.11.2008**
(21) Application number: 06114517.3
(22) Date of filing: 24.05.2006
(51) Int. Cl.: C07D 211/90

(54) **Process for Producing Lercanidipine**
Verfahren zur Herstellung von Lercanipidin
Procédé pour la Preparation de Lercanipidine

(43) Date of publication of application: 28.11.2007
(73) Proprietor: CF Pharma Ltd., 1097 Budapest (HU)
(72) Inventor: Bor, Ádám, 2040, Budaörs (HU); Orosz, György, 1143, Budapest (HU); Szücs, Stefánia Cserépi, 1204, Budapest (HU); Bertolino, Andrea, 1145, Budapest (HU); Lukács, Ferenc, 2143, Kistarcsa (HU); Garaczi, Sándor, 1048, Budapest (HU); Schneider, Géza, 1097, Budapest (HU)
(74) Representative: UEXKÜLL & STOLBERG

(56) References cited:
- EP-A2- 0 153 016
- WO-A-96/35668
- BARANDA ET AL: "Instability of calcium channel antagonists during sample preparation for LC-MS-MS analysis of serum samples" FORENSIC SCIENCE INTERNATIONAL, ELSEVIER SCIENTIFIC PUBLISHERS IRELAND LTD, IE, vol. 156, no. 1, 6 January 2006 (2006-01-06), pages 23-34, XP005239538 ISSN: 0379-0738
- LEONARDI A ET AL: "Asymmetric N-(3,3-diphenylpropyl)aminoalkyl esters of 4-aryl-2,6-dimethyl-1,4-dihydropyridine-3, 5-dicarboxylic acids with antihypertensive activity" EUROPEAN JOURNAL OF MEDICINAL CHEMISTRY, EDITIONS SCIENTIFIQUE ELSEVIER, PARIS, FR, vol. 33, no. 5, June 1998 (1998-06), pages 399-420, XP004127371 ISSN: 0223-5234

## Description

The present invention relates to a process for preparing lercanidipine and its intermediates in high purity.

Lercanidipine (1,4-dihydro-2,6-dimethyl-4-(3-nitrophenyl)-3,5-pyridinedicarboxylic acid 2-[(3,3-diphenylpropyl)methylamino]-1,1-dimethylethyl methyl ester) is a highly lipophilic dihydropyridine calcium antagonist which is suitable for treating hypertension. Lercanidipine has the following Formula I:

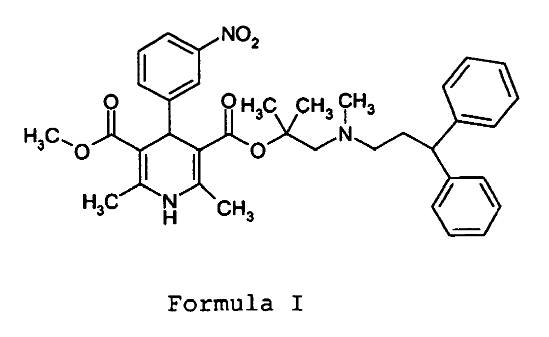

This compound is used in antihypertensive medication in numerous countries since 1997. Like other dihydropyridine-type calcium channel blockers, Lercanidipine causes systemic vasodilation by blocking the influx of calcium ions through L-type calcium channels in cell membranes. Due to its high lipophilicity it has a slower onset and longer duration of action than other calcium antagonists of the dihydropyridine type.

Lercanidipine is commercially available from Recordati S.p.A. (Milan, Italy).

Although Corsini et al., Journal of Cardiovascular Pharmacology, 28(5), 687-694, 1996, showed that the (S)-enantiomer of lercanidipine is more active than the (R)-enantiomer the (R,S) racemate is used in the medicament Zanidip®.

The syntheses of lercanidipine was firstly described in EP 0 153 016 A2. Two alternative routes are disclosed which involve the following reaction schemes:

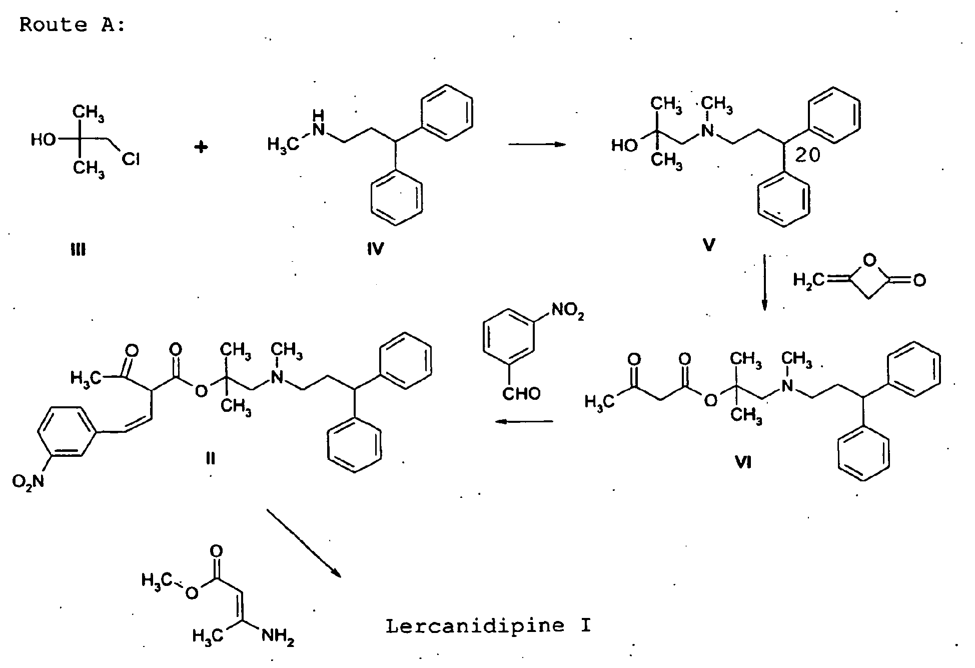

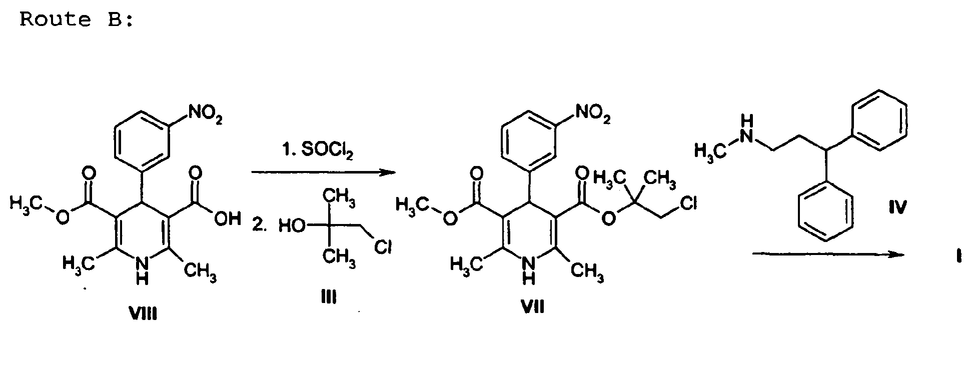

Lercanidipine was isolated in the form of an oily residue which was purified by flash chromatography. Flash chromatography is difficult to apply on an industrial scale. Furthermore, the process reported in EP 0 153 016 A2 results in the formation of side products which causes a low yield of the desired product.

US 5,912,351 describes an alternative process which involves reaction of 1,4-dihydro-2,6-dimethyl-5-methoxycarbonyl-4-(3-nitrophenyl) pyridine-3-carboxylic acid with thionyl chloride in dichloromethane and dimethylformamide and subsequent esterification of the obtained acid chloride with 2,N-dimethyl-N-(3,3-diphenylpropyl)-1-amino-2-propyl alcohol:

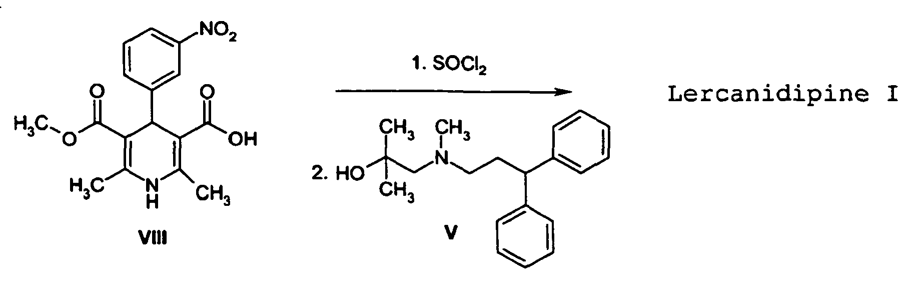

The process is said to yield lercanidipine hydrochloride in an anhydrous non-hygroscopic crystalline form. The product was purified by crystallization to give lercanidipine hydrochloride having a melting point of 186-188 °C, later named Form (C). This process is said to give improved yields and to result in the formation of fewer side products. However, the purity of the product is still not satisfactory and is not reproducible.

WO03/014084 discloses the preparation of various polymorphic forms of lercanidipine hydrochloride, Form (A) having a melting point of 150-152 °C, Form (B) having a melting point of 131-135 °C, Form (I) having a melting point of 197-201 °C and Form (II) having a melting point of 207-211 °C, which are said to be reproducibly obtainable on commercial scale. Forms (A) and (B) were produced from Form (C) by a protocol involving seven process steps. Forms (A) and (B) can be converted to Forms (I) and (II).

WO03/014085 describes the formation of solvates of lercanidipine hydrochloride with various organic solvents, and crystalline forms (III) and (IV) which are obtainable from the solvates by removing the solvation solvent. The solvates and crystalline forms of lercanidipine hydrochloride were prepared by using Forms (I), (A) or (B) as starting material.

WO2006/021397 discloses the preparation of amorphous and crystalline acid addition salts of lercanidipine. These salts are prepared by adding a solution of an acid to a solution of lercanidipine free base followed by the removal of the solvent. Crystalline salts are obtained by recrystallization of the isolated salts from an aprotic and/or protic solvent.

WO2006/046830 discloses a method for preparing amorphous lercanidipine comprising the steps of (1) dissolving lercanidipine in an organic solvent; (2) forming a precipitate by the addition of a second solvent or concentrating the solution under reduced pressure to obtain a solid substance; (3) filtering, washing and drying the precipitate or drying the solid substance.

Although the known processes often require numerous purification steps, none of the known methods is completely satisfactory as far as purity is concerned. It is therefore an object of the present invention to provide a new process for the manufacture of lercanidipine which allows the production of the active ingredient on an industrial scale in high purity and with a minimum number of purification steps.

According to the present invention this problem is surprisingly solved by a process for preparing lercanidipine of formula I, i.e. 1,4-dihydro-2,6-dimethyl-4-(3-nitrophenyl)-3,5-pyridinedicarboxylic acid 2-[(3,3-diphenylpropyl)methylamino]-1,1-dimethylethyl methyl ester, which comprises reacting 1,4-dihydro-2,6-dimethyl-4-(3-nitrophenyl)-3,5-pyridinedicarboxylic acid 1,1-dimethylethyl-2-methylaminoethyl methyl ester IX or a salt thereof with a compound of the general formula XI

L-O-CH₂-CH₂-CHPh₂ XI

wherein L-O is a leaving group.

Compound IX can be used in the form of the free base but is preferably used in the form of an acid addition salt. Preferred acid addition salts are the hydrochloride, tosylate, tartrate, citrate, more preferably the oxalate salt. These salts can be prepared by reaction of the free base of compound IX with the corresponding acid.

The leaving group L-O of compound XI is preferably a triflate (trifluormethane sulfonic), brosylate (4-bromobenzene sulfonic), or napsylate (naphthalensulfonic) group, more preferably a tosylate (4-methyl benzenesulfonic) group. The preparation of compounds according to general formula XI can be achieved by known procedures starting at 3,3-diphenyl propanol. The synthesis of the preferred tosylated compound 3-tosyloxy-3,3-diphenylpropane XIa can be achieved e.g. by tosylating 3,3-diphenyl propanol according to Pitha et al., Collect. Czech. Chem. Commun. 25, 1960, 736-742. 3,3-Diphenyl propanol is accessible e.g. by the method described in DE 766 207.

The reaction of compound IX with compound XI is preferably performed in a solvent selected from the group consisting of toluene, xylene, dimethylformamide (DMF), dimethylacetamide (DMA), hexamethylphosphoric amide (HMPT), N-methyl pyrrolidine and acetonitrile. The most preferred solvent is toluene.

The reaction temperature is preferably within a range of from 10 to 140 °C, more preferably 78 to 82 °C.

It is preferred to perform the reaction in the presence of a base which is preferably selected from the group consisting of alkali carbonates and alkaline-earth carbonates. Preferred alkali metal carbonates are sodium and potassium carbonate, preferred alkaline-earth carbonates are magnesium and calcium carbonate. The most preferred carbonate is potassium carbonate.

It is also preferred to perform the reaction in the presence of a phase transfer catalyst. Preferably the catalyst is selected from the group consisting of tetraalkyl quaternary ammonium salts, more preferably tetra(C₂₋₆)-alkyl quaternary ammonium salts. The most preferred phase transfer catalyst is tetrabutylammonium iodide.

Compound IX used in the above process is preferably prepared by esterification of 1,4-dihydro-2,6-dimethyl-4-(3-nitrophenyl)-3,5-pyridinedicarboxylic acid monomethyl ester VIII with 1,1-dimethyl-2-methylaminoethanol X, or a salt thereof, which can be prepared e.g. by the method described in US 2,252,713. Compound VIII is accessible according to known procedures, e.g. by the method disclosed in DE 28 47 237.

Compound VIII is preferably first halogenated with a halogenation agent to give the acid halide derivative of VIII. Preferably, the halogenation is a chlorination which results in the formation of 1,4-dihydro-2,6-dimethyl-4-(3-nitrophenyl)-3,5-pyridinedicarboxylic acid chloride monomethyl ester. Chlorination can be achieved by using known chlorinating agents such as phosphorus pentachloride, phosphorus trichloride, phosphorus oxychloride, sulfuryl chloride and in particular thionyl chloride. Halogenated compound VIII is then reacted with compound X. The reaction can be performed in situ without prior isolation of halogenated compound VIII.

Alternatively, compound VIII can be activated by the addition of a suitable diimide such as N,N'-dicyclohexylcarbodiimide or by forming a mixed anhydride.

It is preferred to use compound X in the form of its triflate, hydrochloride or sulfate salt, and particularly preferred in the form of its tosylate salt (1,1-dimethyl-2-methylaminoethanol tosylate). It was surprisingly found that e.g. the tosylate salt of compound X can be O-acylated without protection of its amino group. The tosylate salt of X can be prepared from 2-chloro-1,1-dimethylethanol which can be synthesized according to the method described in US 2,252,713. The tosylate salt can be purified and isolated in good yield. The tosylate salt of X is then reacted with compound VIII or preferably halogenated compound VIII to give intermediate IX in pure form and with high yield.

The reaction of compound VIII with compound X is preferably performed in a solvent selected from the group consisting of dimethylformamide (DMF), dimethyl acetamide (DMA) and tetrahydrofuran (THF). The reaction temperature is preferably within a range of from -20 to 30 °C.

The halogenation of compound VIII is preferably performed in a solvent selected from the group consisting of dimethylformamide (DMF), dimethyl acetamide (DMA), tetrahydrofuran (THF), N-methyl pyrrolidine and dimethylether (DME). The reaction temperature is preferably within a range of from -20 to 30 °C.

The process of the present invention for the synthesis of lercanidipine by reaction of compound IX with compound XI results in the formation of the desired product in good yield and in an unexpectedly high purity.

The stereo isomeric form of the product of course depends on the stereo isomeric form of the starting material. If the racemate of compound VIII or IX is used, lercanidipine is also obtained in the form of the racemate. If the (R)-enantiomer or the (S)-enantiomer of compounds VIII or IX is used in the process of the present invention, the (R)-enantiomer or the (S)-enantiomer of lercanidipine can be obtained. The homochiral acids of compound VIII can be prepared by resolution of racemic acid VIII according to the method reported by Ashimori at al., Chem. Pharm. Bull. 39, 108 (1991). It is preferred to produce the (R,S) racemate of I, more preferably the (S)-enantiomer.

The process of the present invention results in the formation of lercanidipine I in the form of the free base. The free base can be converted to an acid addition salt by adding an appropriate acid. Preferably, lercanidipine free base is dissolved in an organic solvent, preferably an ether, more preferably methyl tertiary-butyl ether (MTBE), and an acid or a solution of an acid in a solvent, preferably an organic solvent is added.

Suitable salts of lercanidipine include salts of inorganic acids such as hydrochloric acid, hydrobromic acid, phosphoric acid and sulphuric acid; sulphonic acids such as methanesulphonic acid, benzenesulphonic acid, toluenesulphonic acid, and naphthalene-1,5-disulphonic acid; monocarboxylic acids, such as acetic acid, (+)-L-lactic acid, DL-lactic acid, DL-mandelic acid, gluconic acid, cinnamic acid, salicylic acid, and gentisic acid; dicarboxylic acids, such as oxalic acid, 2-oxo-glutaric acid, malonic acid, (-)-L-malic acid, mucic acid, (+)-L-tartaric acid, fumaric acid, maleic acid, and terephthalic acid; tricarboxylic acids, such as citric acid; and aromatic sulphonimides such as saccharin. The preparation of these addition salts of lercanidipine is disclosed in WO2006/021397.

Preferred salts of lercanidipine are the L-lactate, cinnamate, salicylate, maleate, saccharinate, besylate, napadisylate salts, in particular the oxalate salt and the hydrochloride salt.

Lercanidipine free base and the acid addition salts of lercanidipine can be produced in crystalline form and in amorphous form. Amorphous forms can be prepared by the methods disclosed in WO2006/021397 and WO2006/046830.

Lercanidipine produced by the process of the present invention is preferably isolated by crystallization. Crystallization is preferably carried out by use of water containing hydrochloric acid and sodium chloride as disclosed in EP 0 153 016 A2, or in a mixture of MTBE and isopropanol.

The most preferred salt of lercanidipine is the hydrochloride hemihydrate salt. This salt is preferably obtained by crystallization from water containing hydrochloric acid and sodium chloride to give a product having a melting point of 119-123 °C as disclosed in EP 0 153 016 A2.

Lercanidipine produced according to the method of the present invention can be further purified by converting lercanidipine free base to a first acid addition salt, converting the first addition salt back to the free base, followed by converting the free base to a second acid addition salt. The first acid addition salt is preferably the oxalate, the second addition salt is preferably the hydrochloride. Lercanidipine subjected to this purification sequence has a very high purity and is suitable for the production of pharmaceutical composition without further purification.

For the preparation of pharmaceutical compositions comprising lercanidipine as an active ingredient the lercanidipine prepared by the process of the present invention is mixed with at least one pharmaceutically acceptable excipient, and the mixture of the active ingredient and the one or more excipients is then converted to a pharmaceutical dosage form. Suitable excipients and dosage forms are disclosed e.g. in WO03/014084. Preferred dosage forms are tablets, in particular film coated tablets.

Preferred excipients are pharmaceutically acceptable binders, such as lactose, hydroxypropylmethylcellulose and povidone; disintegrants, such as sodium starch glycolate; lubricants, such as magnesium stearate, magnesium palmitate and magnesium oleate; dispersing and suspending agents such as microcrystalline cellulose; plasticizers, such as polyethylene glycol; colorants, such as ferric oxide(s) and titanium dioxide; and other additives such as talcum. Suitable film forming agents are hydroxypropylmethylcellulose, ethylcellulose and polymethacrylates.

In the following the invention will be described by reference to figures and examples.

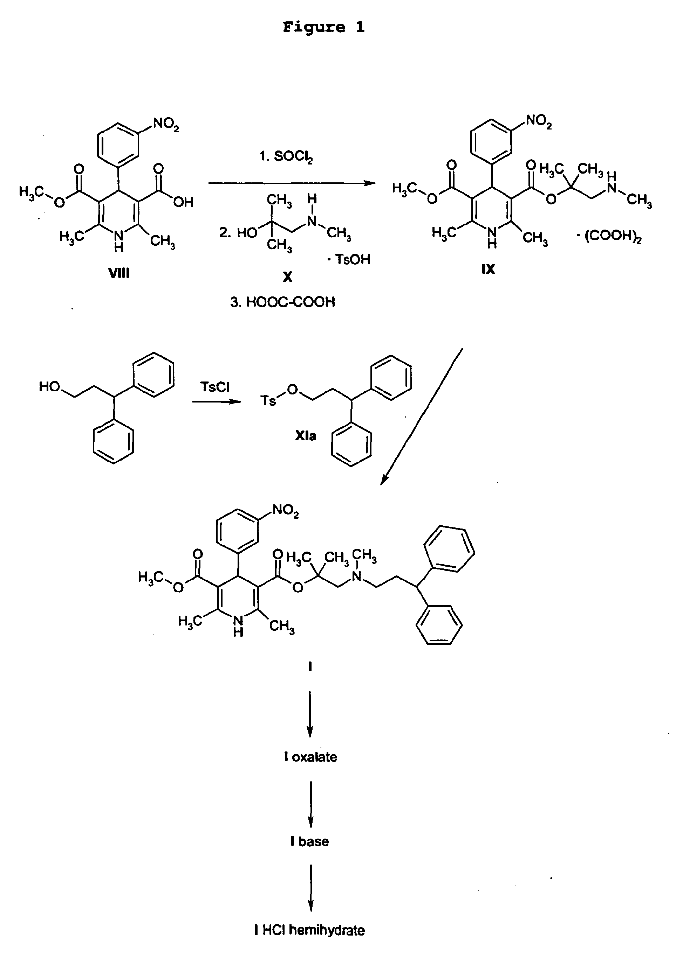

Figure 1 is a reaction scheme showing a preferred embodiment of the process of the present invention. 1,4-Dihydro-2,6-dimethyl-4-(3-nitrophenyl)-3,5-pyridinedicarboxylic acid monomethyl ester VIII is transformed by reaction with thionyl chloride to give 1,4-dihydro-2,6-dimethyl-4-(3-nitrophenyl)-3,5-pyridinedicarboxylic monomethyl ester acid chloride. The acid chloride is then reacted *in situ* with the tosylate salt of 1,1-dimethyl-2-methylaminoethanol X to give 1,4-dihydro-2,6-dimethyl-4-(3-nitrophenyl)-3,5-pyridinedicarboxylic acid 1,1-dimethyl-2-methylaminoethyl methyl ester IX. Alkylation of compound IX with 3-tosyloxy-1,1-diphenylpropane XIa results in the formation of lercanidipine I free base. The free base is purified by forming the oxalate salt which is then converted to the hydrochloride hemihydrate via the free base. The final product is isolated in high yield and with high purity.

### Examples

### Example 1

### 1,1-Dimethyl-2-methylaminoethanol tosylate salt (Formula X)

485 mmol distilled 1,1-dimethyl-2-methylaminoethanol (US 2,252,713; 1938) was dissolved in 50 ml ethyl acetate, then 461. mmol p-toluenesulphonic acid hydrate in 270 ml ethyl acetate was added between 50-60 °C. The solution was cooled to 5-10 °C. The crystalline suspension was filtered. The product was washed with 100 ml cooled ethyl acetate.

| | |
|---|---|
| Yield: | 117.6 g (72.4. %) |
| Purity: | min. 99.5 % |
| Melting point: | 130-132 °C |

### Example 2

### (R,S) 1,4-Dihydro-2,6-dimethyl-4-(3-nitrophenyl)-3,5-pyridinedicarboxylic acid 1,1-dimethyl-2-methylaminoethyl methyl ester oxalate (Formula IX oxalate)

361 mmol (R,S) 1,4-dihydro-2,6-dimethyl-4-(3-nitrophenyl)-3,5-pyridinedicarboxylic acid monomethyl ester (Formula **VIII**) (DE 2,847,237; 1938) was dissolved in a mixture of 600 ml abs. THF and 180 ml abs. DMF. The solution was cooled to -10 to 0 °C and 379 mmol of thionyl chloride was added slowly maintaining the temperature below 0 °C. Following a 1 hour agitation 357.5 mmol 1,1-dimethyl-2-methylaminoethanol tosylate salt (Formula **X**; Example 1) was added. The reaction mixture was stirred for 2-3 hours, then 700 ml water and 700 ml dichloromethane were added. The mixture was cooled below 0 °C and a 40 % aqueous solution of sodium hydroxide was added till pH 10-11. The organic phase was separated. The aqueous phase was extracted with 2 x 300 ml dichloromethane. The combined organic phases were washed with 3 x 300 ml 5 % aqueous solution of sodium hydrogen carbonate. The solution was dried with sodium sulfate and evaporated. The residue was dissolved in 150 ml acetonitrile. 360 mmol oxalic acid dihydrate in 150 ml acetonitrile was added to form the oxalate salt. The crystalline suspension was cooled to 12-15 °C. **IX** oxalate was filtered, washed with 100 ml cooled acetonitrile and dried.

| | |
|---|---|
| Yield: | 154.2 (85 %) |

The product was recrystallized from 450 ml acetonitrile.

| | |
|---|---|
| Yield: | 130 g (71.6 %) |
| Purity: | 99.8 % (HPLC) |
| Melting point : | 161-162 °C |

### Example 3

### 3-Tosyloxy-1,1-diphenylpropane (Formula XI)

1 mol 3,3-diphenylpropanol (DE 766,207; 1940) and 1.34 mol triethylamine were dissolved in 420 ml toluene then 1.1 mol p-toluenesulfonylchloride in 360 ml toluene was added maintaining the temperature between 20-26 °C. The mixture was agitated 5 hours and 400 ml 1N hydrochloric acid was added. The organic phase was separated and washed with 400 ml 5 % sodium hydrogen carbonate solution. The toluenic solution was evaporated. The residue was solved in 1,480 ml ethanol. The solution was cooled to -5 to -10 °C. The crystalline suspension was filtered and washed with 500 ml cooled ethanol.

| | |
|---|---|
| Yield: | 307.4 g (83.9 %) |
| Purity: | 99.9 % (HPLC) |
| Melting point: | 49-51 °C |

### Example 4

### (R,S) 1,4-Dihydro-2,6-dimethyl-4-(3-nitrophenyl)-3,5-pyridinedicarboxylic acid 2-[(3,3-diphenylpropyl)methylamino]-1,1-dimethylethyl methyl ester oxalate (Formula I oxalate)

500 mmol **IX** oxalate salt (Example 2) and 500 mmol **XI** (Example 3) were suspended in 760 ml toluene then 5 mol dried potassium carbonate and 15 g tetrabutylammoniumiodide were added. The mixture was stirred at 78-82 °C for 24 hours in a N₂ stream in the dark while conversion was 55-65 %. The mixture was cooled, then 2,500 ml 0.1 N sodium hydroxide solution and 500 ml toluene were added. The organic phase was separated. The aqueous phase was extracted with 500 ml toluene. The combined organic phases were washed with 500 ml 0.1 N sodium hydroxide solution, 2 x 400 ml 10 % copper sulfate solution and 4 x 500 ml methanol-water mixture (1 : 2). The toluenic solution was extracted with 2 x 400 ml diluted hydrochloric acid - methanol mixture (2 : 1). The combined aqueous phase was treated with 10 % sodium hydroxide solution up to pH 11 and extracted with 2 x 500 ml toluene. The organic phase was evaporated and the residue was dissolved in 1000 ml MTBE. 300 mmol oxalic acid dihydrate in 210 ml isopropanol were added to the solution. 2,100 ml MTBE was cooled to -15 to -20 °C and the solution of the oxalate salt was added maintaining the temperature between -10 to -15 °C. The crystalline suspension was agitated 1 hour at -10 to -15 °C and filtered. The product was washed with 4 x 500 ml cooled MTBE and was dried.

| | |
|---|---|
| Yield: | 181.5 g (51.7 % without recovery of **IX** or 76.1 % with recovery of IX, resp.) |
| Purity: | 99.5 % (HPLC) |
| Melting point: | 120 - 125 °C |

### Recovery of IX oxalate salt

In the above reaction unreacted **IX** was removed by extraction with the aqueous solution of copper sulfate. **IX** was recovered by addition of ammonium hydroxide following extraction. The 800 ml 10 % copper sulfate solution containing unreacted **IX** was treated with NH₄OH solution until the Cu(OH)₂ precipitate was dissolved. The solution was extracted with 4 times 500 ml dichloromethane. The combined organic phases were evaporated. The residue was dissolved in 100 ml acetonitrile. 200 mmol oxalic acid dihydrate in 100 ml acetonitrile was added to form the oxalate salt. The crystalline suspension was cooled to 12 to 15 °C. **IX** oxalate salt was filtered and washed with 60 ml cooled acetonitrile and was dried.

| | |
|---|---|
| Yield: | 81 g (160 mmol) |

### Recovery of XI

The toluenic solution, which was washed with sodium hydroxide, copper-sulfate and methanol-water solutions, was evaporated. The residue was dissolved in 300 ml ethanol. The solution was cooled to -5 to 10 °C. The crystalline suspension was filtered and washed with 100 ml cooled ethanol.

| | |
|---|---|
| Yield: | 54.4 g (148 mmol) |

### Example 5

### (R,S) 1,9-Dihydro-2,6-dimethyl-4-(3-nitrophenyl)-3,5-pyridinedicarboxylic acid 2-[(3,3-diphenylpropyl)methylamino]-1,1-dimethylethyl methyl ester hydrochloride hemihydrate (Formula I HCl ½ H₂O)

10 mmol **I** oxalate (Example 4) was dissolved in 100 ml dichloromethane and 20 mmol potassium carbonate in 20 ml water was added. The organic phase was separated. The aqueous phase was extracted with 2 x 10 ml dichloromethane. The combined organic phases were treated with 20 ml 1N hydrochloric acid. The organic phase was separated and was evaporated. The residue was treated with 2,000 ml of water containing 2 ml of 1N hydrochloride acid and 5 ml of water saturated with sodium chloride to form I hydrochloric hemihydrate, which was filtered.

| | |
|---|---|
| Yield: | 6.2 g (95 %) |
| Purity: | 99.8 % (HPLC) |
| Melting point: | 118-122 °C |
| | |
| HPLC method: | Column RP 18, 125 x 3 mm ID, 5 µm, det: 233 nm |
| Eluent: | 0.01 mol Na₂HPO₄/MeOH/ can : 25/65/10 tᵣ ~ 15 min. |

IR, X-ray and DSC proved that the product was identical to the product which was prepared by the process described in Example 16 of EP 0 153 016 A2.

## Claims

1. A process for preparing 1,4-dihydro-2,6-dimethyl-4-(3-nitrophenyl)-3,5-pyridinedicarboxylic acid 2-[(3,3-diphenylpropyl)methylamino]-1,1-dimethylethyl methyl ester of formula I, or a salt thereof, 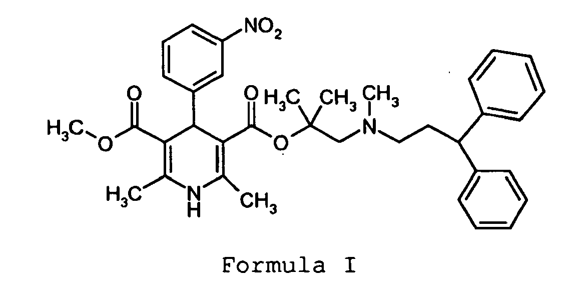 which comprises reacting 1,4-dihydro-2,6-dimethyl-4-(3-nitrophenyl)-3,5-pyridinedicarboxylic acid 1,1-dimethylethyl-2-methylaminoethyl methyl ester of formula IX, or a salt thereof, 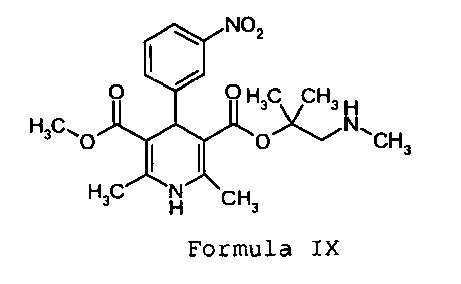 with a compound of the general formula XI
L-O-CH₂-CH₂-CHPh₂ XI
wherein L-O is a leaving group.

2. The process of claim 1, wherein the salt of compound IX is the oxalate, tosylate, citrate, tartrate salt.

3. The process of claim 1 or 2, wherein the leaving group L-O is a tosylate, triflate, brosylate or napsylate group.

4. The process of any one of the preceding claims, wherein the reaction is conducted in a solvent selected from the groups consisting of toluene, xylene, dimethylformamide (DMF), dimethylacetamide (DMA), hexamethylphosphoric amide (HMPT), N-methyl pyrrolidine and acetonitrile.

5. The process of any one of the preceding claims, wherein the reaction is performed at a temperature of 10 to 140 °C.

6. A process for preparing 1,4-dihydro-2,6-dimethyl-4-(3-nitrophenyl)-3,5-pyridinedicarboxylic acid 1,1-dimethylethyl-2-methylaminoethyl methyl ester of formula IX, or a salt thereof, 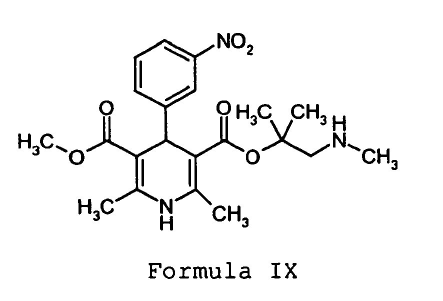 wherein 1,4-dihydro-2,6-dimethyl-4-(3-nitrophenyl)-3,5-pyridinedicarboxylic acid monomethyl ester of formula VIII, or an acid halide thereof, 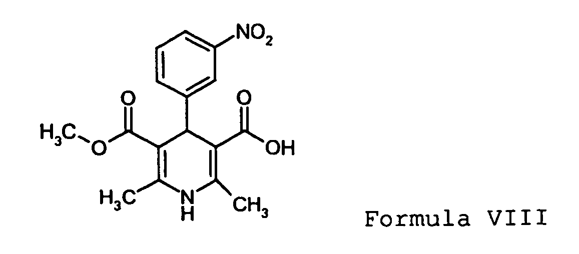 is reacted with 1,1-dimethyl-2-methylaminoethanol of formula X, or a salt thereof,

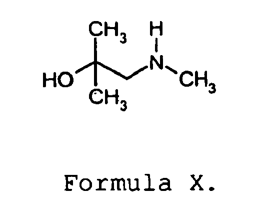

7. The process of claim 6, comprising halogenating compound VIII to give 1,4-dihydro-2,6-dimethyl-4-(3-nitrophenyl)-3,5-pyridinedicarboxylic acid halide monomethyl ester and reacting the acid halide of VIII with compound X or a salt thereof.

8. The process of claim 7, wherein the halogenating agent is thionyl chloride, phosphorus pentachloride, phosphorus trichloride, phosphorus oxychloride or sulfuryl chloride.

9. The process of any one of claims 6 to 8, wherein compound X is used in the form of its triflate, hydrochloride, sulfate or tosylate salt.

10. The process of claim 6, wherein the reaction is performed in a solvent selected from the group consisting of THF, DMF and DMA.

11. The process of claim 6 or 10, wherein the reaction is performed at a temperature of -20 to 30 °C.

12. The process of claim 7 or 8, wherein the reaction is performed in a solvent selected from the groups consisting of DMF, THF, DME, N-methylpyrrolidine.

13. The process of claim 7, 8 or 12, wherein the reaction is performed at a temperature of -20 to 30 °C.

14. The process of any one of the preceding claims, further comprising converting the free base of compound I to a first acid addition salt, converting the first addition salt back to the free base, followed by converting the free base to a second acid addition salt.

15. The process of claim 14, wherein the first acid addition salt is the oxalate.

16. The process of claim 14 or 15, wherein the second addition salt is the hydrochloride hemihydrate.

17. The process of any one of the preceding claims, further comprising isolating the resultant product.

18. The process of claim 17, wherein the product is isolated by crystallization.

19. The process of claim 17, wherein the product is isolated in amorphous form.

20. An acid addition salt of 1,4-Dihydro-2,6-dimethyl-4-(3-nitrophenyl)-3,5-pyridinedicarboxylic acid 1,1-dimethylethyl-2-methylaminoethyl methyl ester of formula IX:

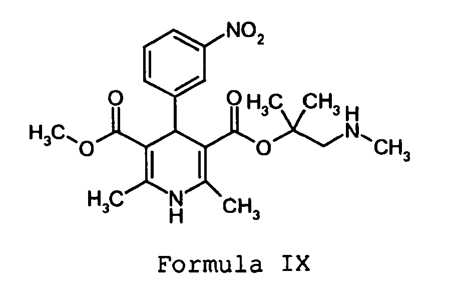

21. The compound of claim 20, which is the tosylate, tartrate, citrate, or oxalate salt.

22. The use of 1,4-Dihydro-2,6-dimethyl-4-(3-nitrophenyl)-3,5-pyridinedicarboxylic acid 1,1-dimethylethyl-2-methylaminoethyl methyl ester of formula IX, or a salt thereof: 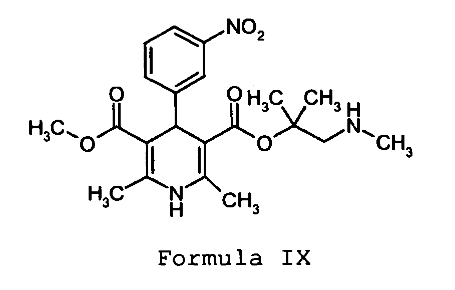 for manufacturing 1,4-dihydro-2,6-dimethyl-4-(3-nitrophenyl)-3,5-pyridinedicarboxylic acid 2-[(3,3-diphenylpropyl)methylamino]-1,1-dimethylethyl methyl ester of formula I.

23. The use of claim 22, wherein the salt is acid addition salt.

24. The use of claim 23, wherein the salt is the tosylate, tartrate, citrate, or oxalate salt.

## Patentansprüche

1. Verfahren zur Herstellung von 1,4-Dihydro-2,6-dimethyl-4-(3-nitrophenyl)-3,5-pyridindicarbonsäure-2-[(3,3-diphenylpropyl)methylamino]-1,1-dimethylethylmethylester der Formel I oder eines Salzes davon, bei dem 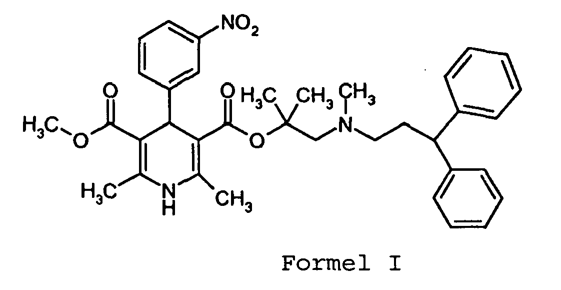 man 1,4-Dihydro-2,6-dimethyl-4-(3-nitrophenyl)-3,5-pyridindicarbonsäure-1,1-dimethylethyl-2-methylaminoethylmethylester der Formel IX oder ein Salz davon, 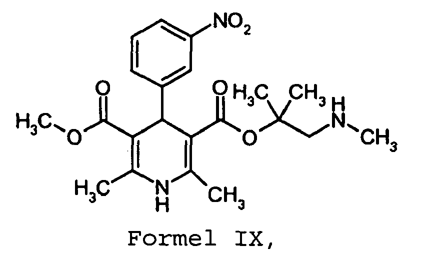 mit einer Verbindung mit der Formel XI:
L-O-CH₂-CH₂-CHPh₂ XI
umsetzt, wobei L-O eine Abgangsgruppe ist.

2. Verfahren nach Anspruch 1, bei dem das Salz der Verbindung IX das Oxalat-, Tosylat-, Citrat- und/oder Tartratsalz ist.

3. Verfahren nach Anspruch 1 oder 2, bei dem die Abgangsgruppe L-O eine Tosylat-, Triflat-, Brosylat- oder Napsylatgruppe ist.

4. Verfahren nach einem der vorhergehenden Ansprüche, bei dem die Reaktion in einem Lösungsmittel ausgewählt aus der Gruppe bestehend aus Toluol, Xylol, Dimethylformamid (DMF), Dimethylacetamid (DMA), Hexamethylphosphorsäureamid (HMPT), N-Methylpyrrolidin und Acetonitril durchgeführt wird.

5. Verfahren nach einem der vorhergehenden Ansprüche, bei dem man die Reaktion bei einer Temperatur von 10 bis 140°C durchführt.

6. Verfahren zur Herstellung von 1,4-Dihydro-2,6-dimethyl-4-(3-nitrophenyl)-3,5-pyridindicarbonsäure-1,1-dimethylethyl-2-methylaminoethylmethylester der Formel IX oder eines Salzes davon, 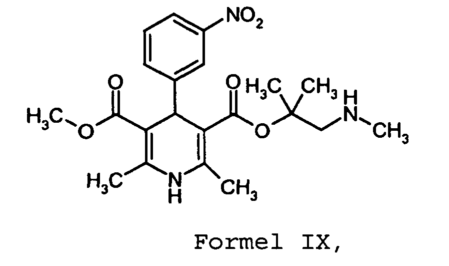 wobei man 1,4-Dihydro-2,6-dimethyl-4-(3-nitrophenyl)-3,5-pyridindicarbonsäuremonomethylester der Formel VIII oder ein Säurehalogenid davon, 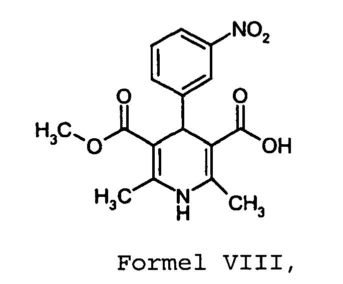 mit 1,1-Dimethyl-2-methylaminoethanol der Formel X oder einem Salz davon umsetzt,

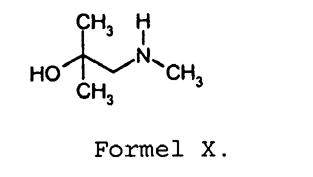

7. Verfahren nach Anspruch 6, bei dem man Verbindung VIII unter Bildung von 1,4-Dihydro-2,6-dimethyl-4-(3-nitrophenyl)-3,5-pyridindicarbonsäurehalogenidmonomethylester halogeniert und man das Säurehalogenid von VIII mit Verbindung X oder einem Salz davon umsetzt.

8. Verfahren nach Anspruch 7, bei dem das Halogenierungsmittel Thionylchlorid, Phosphorpentachlorid, Phosphortrichlorid, Phosphoroxychlorid oder Sulfurylchlorid ist.

9. Verfahren nach einem der Ansprüche 6 bis 8, bei dem Verbindung X in Form ihres Triflat-, Hydrochlorid-, Sulfat- oder Tosylatsalzes verwendet wird.

10. Verfahren nach Anspruch 6, bei dem man die Reaktion in einem Lösungsmittel ausgewählt aus der Gruppe bestehend aus THF, DMF und DMA durchgeführt.

11. Verfahren nach Anspruch 6 oder 10, bei dem man die Reaktion bei einer Temperatur von -20°C bis 30°C durchführt.

12. Verfahren nach Anspruch 7 oder 8, bei dem man die Reaktion in einem Lösungsmittel ausgewählt aus der Gruppe bestehend aus DMF, THF, DME, N-Methylpyrrolidin durchgeführt.

13. Verfahren nach Anspruch 7, 8 oder 12, bei dem man die Reaktion bei einer Temperatur von -20°C bis 30°C durchführt.

14. Verfahren nach einem der vorhergehenden Ansprüchen, bei dem man ferner die freie Base von Verbindung I in ein erstes Säureadditionssalz überführt, man das erste Additionssalz wieder in die freie Base umwandelt und man anschließend die freie Base in ein zweites Säureadditionssalz überführt.

15. Verfahren nach Anspruch 14, bei dem das erste Säureadditionssalz das Oxalat ist.

16. Verfahren nach Anspruch 14 oder 15, bei dem das zweite Säureadditionssalz das Hydrochloridhemihydrat ist.

17. Verfahren nach einem der vorhergehenden Ansprüche, bei dem man ferner das resultierende Produkt isoliert.

18. Verfahren nach Anspruch 17, bei dem man das Produkt durch Kristallisation isoliert.

19. Verfahren nach Anspruch 17, bei dem man das Produkt in amorpher Form isoliert.

20. Säureadditionssalz von 1,4-Dihydro-2,6-dimethyl-4-(3-nitrophenyl)-3,5-pyridindicarbonsäure-1,1-dimethylethyl-2-methylaminoethylmethylester der Formel IX:

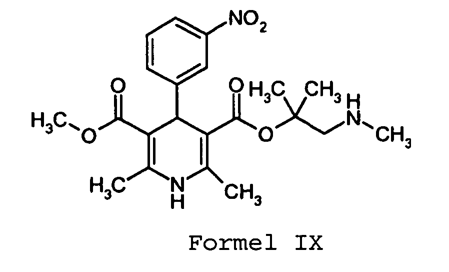

21. Verbindung nach Anspruch 20, die das Tosylat-, Tartrat-, Citrat- oder Oxalatsalz ist.

22. Verwendung von 1,4-Dihydro-2,6-dimethyl-4-(3-nitrophenyl)-3,5-pyridindicarbonsäure-1,1-dimethylethyl-2-methylaminoethylmethylester der Formel IX oder eines Salzes davon, 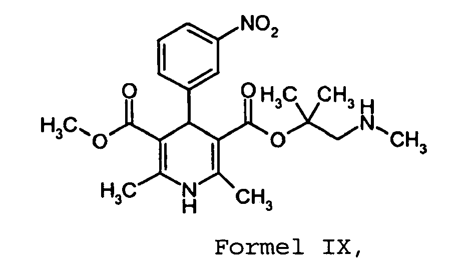 zur Herstellung von 1,4-Dihydro-2,6-dimethyl-4-(3-nitrophenyl)-3,5-pyridindicarbonsäure-2-[(3,3-diphenylpropyl)-methylamino]-1,1-dimethylethylmethylester der Formel I.

23. Verwendung nach Anspruch 22, bei der das Salz ein Säureadditionssalz ist.

24. Verwendung nach Anspruch 23, bei der das Salz das Tosylat-, Tartrat-, Citrat- oder Oxalatsalz ist.

## Revendications

1. Procédé de préparation du 1,4-dihydro-2,6-diméthyl-4-(3-nitrophényl)-pyridine-3,5-dicarboxylate de méthyle et de 2-[(3,3-diphénylpropyl)-méthylamino]-1,1-diméthyl-éthyle, composé de formule I, ou d'un sel de ce composé : 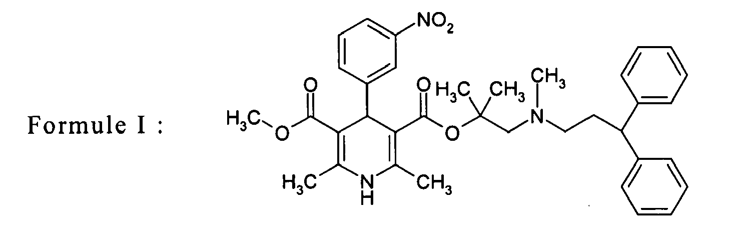 lequel procédé comporte le fait de faire réagir du 1,4-dihydro-2,6-diméthyl-4-(3-nitro-phényl)-pyridine-3,5-dicarboxylate de méthyle et de 1,1-diméthyl-2-méthylamino-éthyle, composé de formule IX, ou un sel de ce composé : 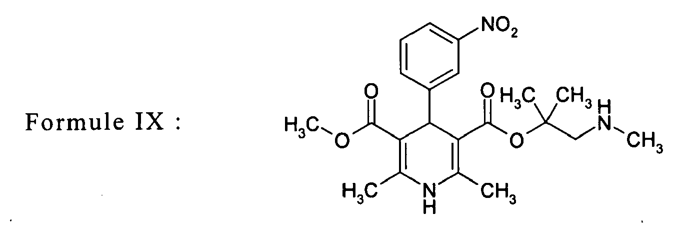 avec un composé de formule générale XI :
L-O-CH₂-CH₂-CHPh₂ XI
dans laquelle L-O représente un groupe partant.

2. Procédé conforme à la revendication 1, dans lequel le sel du composé de formule IX est un oxalate, un tosylate, un citrate ou un tartrate de ce composé.

3. Procédé conforme à la revendication 1 ou 2, dans lequel le groupe partant représenté par L-O est un groupe tosylate, triflate, brosylate ou napsylate.

4. Procédé conforme à l'une des revendications précédentes, dans lequel on effectue la réaction dans un solvant choisi dans l'ensemble formé par les suivants : toluène, xylène, diméthyl-formamide (DMF), diméthyl-acétamide (DMA), hexaméthylphosphotriamide (HMPT), N-méthyl-pyrrolidine et acétonitrile.

5. Procédé conforme à l'une des revendications précédentes, dans lequel on effectue la réaction à une température de 10 à 140 °C.

6. Procédé de préparation du 1,4-dihydro-2,6-diméthyl-4-(3-nitrophényl)-pyridine-3,5-dicarboxylate de méthyle et de 1,1-diméthyl-2-méthylamino-éthyle, composé de formule IX, ou d'un sel de ce composé : 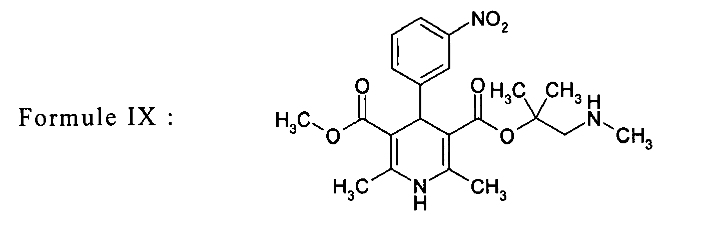 dans lequel on fait réagir du monoester méthylique d'acide 1,4-dihydro-2,6-diméthyl-4-(3-nitro-phényl)-pyridine-3,5-dicarboxylique, composé de formule VIII, ou un halogénure d'acide de ce composé : 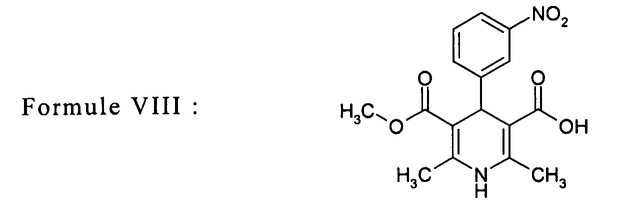 avec du 1,1-diméthyl-2-méthylamino-éthanol, composé de formule X, ou un sel de ce composé :

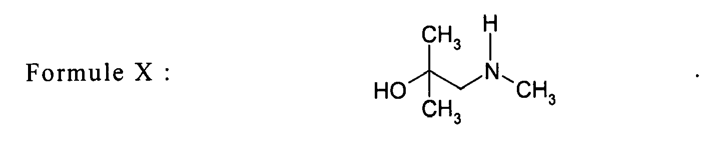

7. Procédé conforme à la revendication 6, qui comporte le fait d'effectuer une halogénation du composé de formule VIII, de manière à obtenir un halogénure de monoester méthylique d'acide 1,4-dihydro-2,6-diméthyl-4-(3-nitro-phényl)-pyridine-3,5-dicarboxylique, et le fait de faire réagir cet halogénure d'acide du composé de formule VIII avec du composé de formule X ou un sel de celui-ci.

8. Procédé conforme à la revendication 7, dans lequel l'agent utilisé pour l'halogénation est du chlorure de thionyle, du pentachlorure de phosphore, du trichlorure de phosphore, de l'oxychlorure de phosphore ou du chlorure de sulfuryle.

9. Procédé conforme à l'une des revendications 6 à 8, dans lequel on utilise le composé de formule X sous forme de sel, à savoir son triflate, son chlorhydrate, son sulfate ou son tosylate.

10. Procédé conforme à la revendication 6, dans lequel on effectue la réaction dans un solvant choisi dans l'ensemble constitué par du tétrahydrofurane (THF), du diméthyl-formamide (DMF) et du diméthyl-acétamide (DMA).

11. Procédé conforme à la revendication 6 ou 10, dans lequel on effectue la réaction à une température de -20 à 30 °C.

12. Procédé conforme à la revendication 7 ou 8, dans lequel on effectue la réaction dans un solvant choisi dans l'ensemble formé par du tétrahydrofurane (THF), du diméthyl-formamide (DMF), du diméthyl-éther (DME) ou de la N-méthyl-pyrrolidine.

13. Procédé conforme à la revendication 7, 8 ou 12, dans lequel on effectue la réaction à une température de -20 à 30 °C.

14. Procédé conforme à l'une des revendications précédentes, qui comporte en outre le fait de convertir le composé de formule I de l'état de base libre en celui d'un premier sel d'addition d'acide, le fait de reconvertir ce premier sel d'addition en la base libre, puis le fait de convertir cette base libre en un deuxième sel d'addition d'acide.

15. Procédé conforme à la revendication 14, dans lequel le premier sel d'addition d'acide est l'oxalate.

16. Procédé conforme à la revendication 14 ou 15, dans lequel le deuxième sel d'addition d'acide est le chlorhydrate hémihydraté.

17. Procédé conforme à l'une des revendications précédentes, qui comporte en outre le fait d'isoler le produit résultant.

18. Procédé conforme à la revendication 17, dans lequel on isole le produit en le faisant cristalliser.

19. Procédé conforme à la revendication 17, dans lequel on isole le produit sous forme amorphe.

20. Sel d'addition d'acide du 1,4-dihydro-2,6-di-méthyl-4-(3-nitrophényl)-pyridine-3,5-dicarboxylate de méthyle et de 1,1-diméthyl-2-méthylamino-éthyle, composé de formule IX :

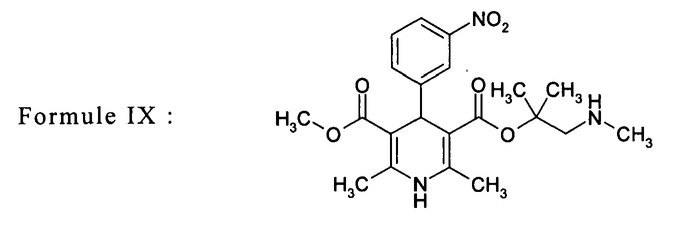

21. Composé conforme à la revendication 20, qui est le sel tosylate, tartrate, citrate ou oxalate.

22. Emploi du 1,4-dihydro-2,6-diméthyl-4-(3-nitro-phényl)-pyridine-3,5-dicarboxylate de méthyle et de 1,1-diméthyl-2-méthylamino-éthyle, composé de formule IX, ou d'un sel de ce composé : 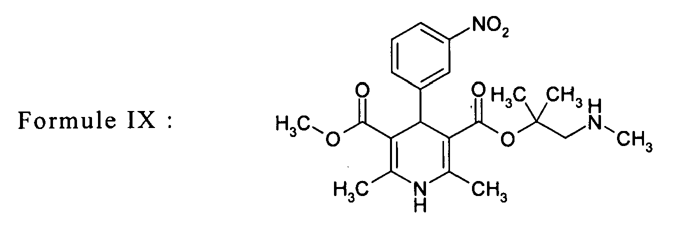 dans la préparation du 1,4-dihydro-2,6-diméthyl-4-(3-nitro-phényl)-pyridine-3,5-dicarboxylate de méthyle et de 2-[(3,3-diphényl-propyl)-méthylamino]-1,1-diméthyl-éthyle, composé de formule I :

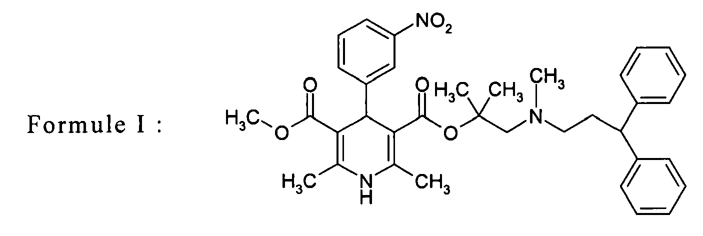

23. Emploi conforme à la revendication 22, dans lequel le sel est un sel d'addition d'acide.

24. Emploi conforme à la revendication 23, dans lequel le sel est le sel tosylate, tartrate, citrate ou oxalate.
